(19) **Europäisches Patentamt**
**European Patent Office**
**Office européen des brevets**

(11) **EP 2 886 058 A1**

(12) **EUROPEAN PATENT APPLICATION**

(43) Date of publication:
24.06.2015 Bulletin 2015/26

(51) Int Cl.:
*A61B 8/06* (2006.01)    *A61B 8/08* (2006.01)

(21) Application number: 14191024.0

(22) Date of filing: 30.10.2014

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: 18.12.2013 KR 20130158675

(71) Applicant: **Samsung Medison Co., Ltd.
Gangwon-do 250-870 (KR)**

(72) Inventor: **Lee, Jin-yong
Hongcheon-gun,
Gangwon-do (KR)**

(74) Representative: **Frey, Sven Holger
LORENZ & KOLLEGEN
Patentanwälte Partnerschaftsgesellschaft mbH
Alte Ulmer Strasse 2
89522 Heidenheim (DE)**

(54) **APPARATUS AND METHOD FOR DISPLAYING A DEGREE OF STENOSIS IN AN ULTRASOUND IMAGE**

(57)    An apparatus and method of displaying an ultrasound image are disclosed, wherein ultrasound data regarding a target object including a blood vessel (300) is obtained. The data may include Doppler data. Blood flow velocities (V1, V2) passing through a plurality of cross-sections (301, 302) of the blood vessel are determined, and stenosis degrees of the plurality of cross-sections are determined based on the blood flow velocities and a continuity equation. The cross-section with the slowest blood flow velocity may be assumed to be free of plaque and may be selected as a reference cross-section. An ultrasound image is generated and displayed, which shows the determined stenosis degrees.

FIG. 3

**Description**

RELATED APPLICATIONS

This application claims the benefit of Korean Patent Application No. 10-2013-0158675, filed on December 18, 2013, in the Korean Intellectual Property Office, the disclosure of which is incorporated herein in its entirety by reference.

BACKGROUND

1. Field

One or more embodiments of the present invention relate to a method and an apparatus for displaying an ultrasound image, and more particularly, to a method and an apparatus for determining a stricture of a blood vessel based on the speed of blood flow in the blood vessel and generating and displaying an ultrasound image showing the determined stricture of the blood vessel.

2. Description of the Related Art

An ultrasonography system is non-invasive and non-destructive, and thus, it is widely used in various medical fields for obtaining information regarding the interior of a target object. A medical practitioner may use the ultrasonography system to acquire high resolution images of tissues inside the target object without any surgery for actually cutting the target object.

Generally, the ultrasonography system transmits ultrasound signals to the target object and receives ultrasound signals reflected by the target object (referred to hereinafter as 'echo signals') while a probe is brought into contact with a surface of the target object. The ultrasonography system generates an ultrasound image of a target object based on echo signals received via the probe and displays the generated ultrasound image via a display.

The ultrasonography system may generate and display a B mode (brightness mode) image, in which intensities of echo signals reflected by a target object are indicated as brightness, or a D mode (Doppler mode) image, in which Doppler components extracted from the echo signals are indicated as colors or waveforms.

Meanwhile, circulatory diseases, such as arteriosclerosis, angina, and myocardial infarction (MI), are often caused by a stenosis degree of a blood vessel. Therefore, to predict a risk for developing a circulatory disease, a precise diagnosis of a stenosis degree of a blood vessel is demanded.

Coronary angiography is used as a method for measuring a stenosis degree of a blood vessel. However, the coronary angiography is an expensive and invasive imaging method. Therefore, an ultrasonography system has been used recently for detecting and measuring a stenosis degree of a blood vessel.

Generally, a blood vessel is measured manually by using an ultrasonography system to obtain a B mode image of a cross-section of a blood vessel, setting an area corresponding to a blood vessel wall based on a user input with respect to the B mode image, and measuring a thickness of the blood vessel wall or area of the blood vessel in the B mode image. However, it is difficult to find out the overall stenosis degrees of the blood vessel based on the image of one cross-section of the blood vessel and diagnosis accuracy may vary from one user to another.

Furthermore, it is easy to recognize fat plagues in the B mode image, but it is difficult to recognize fiber plagues. Therefore, a stenosis degree of a blood vessel caused by accumulation of fiber plagues on a blood vessel wall may not be precisely measured via the B mode image.

SUMMARY

One or more embodiments of the present invention include a method and an apparatus for providing an ultrasound image showing the overall stenosis degrees of a blood vessel having a designated length for precise measurement of the stenosis degrees thereof.

One or more embodiments of the present invention include a method and an apparatus for precisely measuring a stenosis degree of a blood vessel, even when recognition of the stenosis degree in a B mode image is difficult, by determining the stenosis degree of the blood vessel based on a speed of blood flow in the blood vessel and providing an ultrasound image showing the determined stenosis degree of the blood vessel.

Additional aspects will be set forth in part in the description which follows and, in part, will be apparent from the description, or may be learned by practice of the presented embodiments.

According to one or more embodiments of the present invention, a method of displaying an ultrasound image, the method includes obtaining ultrasound data regarding a target object including a blood vessel; obtaining, from the ultrasound data, speeds of blood flows passing through a plurality of cross-sections of the blood vessel; determining stenosis degrees of the plurality of cross-sections based on the blood flow speeds; and generating and displaying an ultrasound image of the blood vessel showing the determined stenosis degrees.

The obtaining of the ultrasound data includes transmitting a planewave ultrasound signal to the target object; receiving ultrasound echo signals reflected by the target object; and obtaining the ultrasound data from the received ultrasound echo signals.

The obtained ultrasound data includes ultrasound Doppler data regarding the target object, and the obtaining of the speeds of the blood flows is performed with respect to the centers of the plurality of cross-sections of the blood vessel.

The determining of the stenosis degrees of the plurality of cross-sections includes selecting a reference cross-section from among the plurality of cross-sections; and determining stenosis degrees of the cross-sections other than the selected reference cross-section based on the speed of the blood flow passing through the selected reference cross-section.

The determining of the stenosis degrees of the plurality of cross-sections includes selecting a reference cross-section from among the plurality of cross-sections; calculating a ratio between an area of the selected reference cross-section and areas of cross-sections other than the selected reference cross-section based on ratios between the speed of the blood flow passing through the selected reference cross-section and the speeds of the blood flows passing through the cross-sections other than the selected reference cross-section; and determining stenosis degrees of the plurality of cross-sections based on the calculated ratios.

The selected reference cross-section is a cross-section corresponding to the slowest blood flow speed from among the plurality of cross-sections or a cross-section based on a user input.

The selecting of the reference cross-section from among the plurality of cross-sections includes generating a B mode image or a C mode image by processing the obtained ultrasound data; and analyzing the B mode image or the C mode image to select the reference cross-section from among the plurality of cross-sections.

The generating and displaying of the image of the blood vessel includes generating an image of the blood vessel that shows the stenosis degrees of the plurality of cross-sections by using at least one from among colors, graphs, and geometric figures.

The generating and displaying of the image regarding the blood vessel includes generating a B mode image showing the blood vessel; displaying the B mode image; and displaying a C mode image in an area of the B mode image inside the blood vessel and displaying colors showing the determined stenosis degrees in an area of the B mode image corresponding to a blood vessel wall of the blood vessel.

According to one or more embodiments of the present invention, an ultrasound image displaying device includes an ultrasound data obtainer, which obtains ultrasound data regarding a target object including a blood vessel; a processor, which obtains speeds of blood flows passing through a plurality of cross-sections of the blood vessel from the ultrasound data, determines stenosis degrees of the plurality of cross-sections based on the blood flow speeds, and generates an ultrasound image of the blood vessel showing the determined stenosis degrees; and a display, which displays the image of the blood vessel.

The ultrasound data obtainer includes an ultrasound probe, which transmits planewave ultrasound signals to the target object and receives ultrasound echo signals reflected by the target object; and a data generator, which obtains the ultrasound data from the received ultrasound echo signals.

The obtained ultrasound data includes ultrasound Doppler data regarding the target object, and the processor obtains speeds of the blood flows passing through the centers of the plurality of cross-sections of the blood vessel.

The processor selects a reference cross-section from among the plurality of cross-sections, and the processor determines stenosis degrees of the cross-sections other than the selected reference cross-section based on a speed of the blood flow passing through the selected reference cross-section.

The processor selects a reference cross-section from among the plurality of cross-sections, and the processor calculates a ratio between area of the selected reference cross-section and areas of cross-sections other than the selected reference cross-section based on ratios between a speed of the blood flow passing through the selected reference cross-section and speeds of the blood flows passing through the cross-sections other than the selected reference cross-section and determines stenosis degrees of the plurality of cross-sections based on the calculated ratios.

The selected cross-section is a cross-section corresponding to the slowest blood flow speed from among the plurality of cross-sections or a cross-section based on a user input.

The processor generates a B mode image or a C mode image by processing the obtained ultrasound data, and the processor analyzing the B mode image or the C mode image to select the reference cross-section from among the plurality of cross-sections.

The processor generates an image of the blood vessel that shows the stenosis degrees of the plurality of cross-sections by using at least one from among colors, graphs, and geometric figures.

The processor generates a B mode image of the blood vessel, the display displays the B mode image, and the display displays a C mode image in an area of the B mode image inside the blood vessel and displays colors showing the determined stenosis degrees in an area of the B mode image corresponding to a blood vessel wall of the blood vessel.

According to one or more embodiments of the present invention, there is provided a computer readable recording medium having recording thereon a computer program for implementing the above-stated method.

BRIEF DESCRIPTION OF THE DRAWINGS

These and/or other aspects will become apparent and more readily appreciated from the following description of the embodiments, taken in conjunction with the accompanying drawings in which:

FIG. 1 is a block diagram showing an ultrasound image displaying device according to an embodiment of the present invention;
FIG. 2 is a flowchart showing a method of displaying an ultrasound image according to an embodiment of the present invention;
FIG. 3 is a diagram for describing a continuity equation employed in a method of displaying an ultrasound image according to an embodiment of the present invention;
FIG. 4A - 4C is a diagram showing an example of images of a blood vessel according to an embodiment of the present invention;
FIG. 5 is a diagram showing an example of images of a blood vessel according to an embodiment of the present invention; and
FIG. 6 is a block diagram showing an ultrasonography system using a method and an apparatus for displaying an ultrasound image according to an embodiment of the present invention.

DETAILED DESCRIPTION

Reference will now be made in detail to embodiments, examples of which are illustrated in the accompanying drawings. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. In the description of the present invention, certain detailed explanations of related art are omitted when it is deemed that they may unnecessarily obscure the essence of the invention. Like reference numerals refer to like elements throughout.

It will be understood that when an element or layer is referred to as being "connected" to another element or layer, the element or layer can be "directly connected" to another element or layer or "electrically connected" across elements. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The terminology "target object" may refer to a living organism or an object to be imaged. Furthermore, the term "target object" may refer to a part of a human body. For example, examples of the target body may include organs, such as a liver, a heart, a uterus, a brain, and a stomach, or a fetus and may include an arbitrary cross-section of a human body. The terminology "user" may be a medical expert, such as a doctor, a nurse, a medical technologist, a sonographer, and a medical image expert, but is not limited thereto.

The terminology "ultrasound image" refers to an image regarding a target object obtained by using ultrasound waves throughout. The terminology "ultrasound data" may refer to data generated based on received signals obtained by transmitting ultrasounds to a target object, receiving echo signals reflected by the target object.

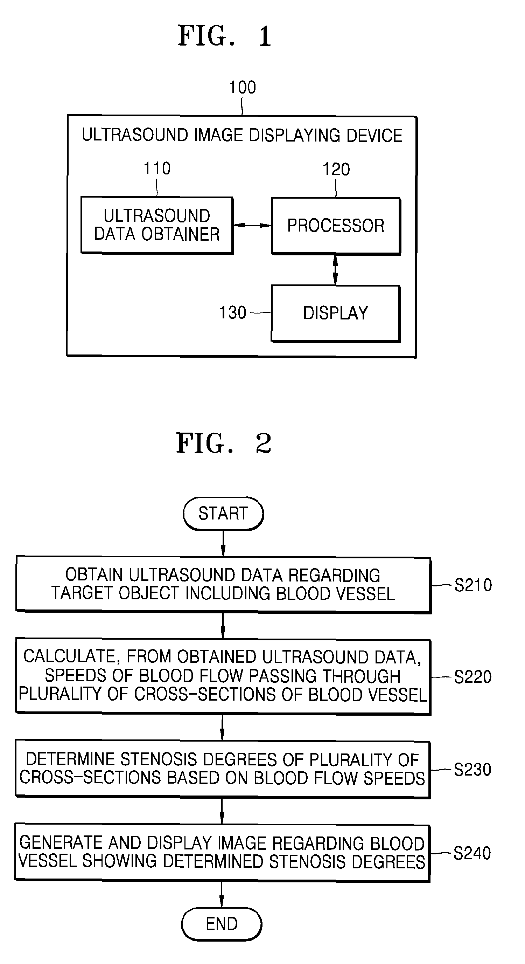

FIG. 1 is a block diagram showing an ultrasound image displaying device according to an embodiment of the present invention.

As shown in FIG. 1, the ultrasound image displaying device 100 includes an ultrasound data obtainer 110, a processor 120, and a display 130.

The ultrasound image displaying device 100 according to an embodiment of the present invention may be a cart-type or a portable type. Examples of portable ultrasound diagnosis devices may include a PACS viewer, a smart phone, a laptop computer, a personal digital assistant (PDA), and a tablet PC. However, the present invention is not limited thereto.

The ultrasound data obtainer 110 obtains ultrasound data regarding a target object including a blood vessel.

For example, the ultrasound data obtainer 110 includes an ultrasound probe which directly transmits and receives ultrasound signals to and from a target object, thereby generating ultrasound data.

The ultrasound probe may transmit ultrasound signals to the target object and receive echo signals reflected by the target object. The ultrasound probe may transmit ultrasound signals to the target object and receive echo signals reflected by the target object according to a driving signal applied to the ultrasound probe.

The ultrasound probe includes a plurality of transducers and the plurality of transducers vibrate according to transmitted electric signals and generate acoustic energy, that is, ultrasound waves. Furthermore, the ultrasound probe may be connected to the main body of the ultrasound image displaying device 100 via a wire or wirelessly The ultrasound image displaying device 100 may include a plurality of ultrasound probes according to embodiments. The ultrasound probe according to an embodiment of the present invention may include at least one from among 1-dimensional (1 D) probe, a 1.5-dimensional (1.5D) probe, a 2-dimensional (2D) (matrix) probe, and a 3-dimensional (3D) probe.

An ultrasound signal transmitted from the ultrasound probe is a non-focal ultrasound signal or a focal ultrasound signal. In other words, an ultrasound signal (ultrasound beam) transmitted from the ultrasound probe includes a general focal ultrasound beam of which focal point is located inside an imaging area, a broad ultrasound beam of which focal point is located outside an imaging area, a planewave ultrasound beam of which focal point is located at the infinity, and a virtual apex ultrasound beam of which focal point is located behind a plane of the ultrasound probe.

The ultrasound probe according to an embodiment of the present invention may transmit planewave ultrasound signals to the target object and may receive ultrasound echo signals reflected by the target object based on the transmitted ultrasound signals. A method of quickly obtaining Doppler data regarding a large area of the target object by using planewave ultrasound signals is referred to as an ultrafast Doppler imaging method.

The ultrasound data obtainer 110 may further include a data generator for obtaining ultrasound data from ultrasound echo signals received at the ultrasound probe.

As another example, the ultrasound data obtainer 110 may receive ultrasound data regarding the target object from the outside. The ultrasound data obtainer 110 may generate ultrasound data from an external device connected to the ultrasound image displaying device 100 via a wire or wirelessly.

Meanwhile, ultrasound data obtained by the ultrasound data obtainer 110 may include ultrasound Doppler data regarding the target object. Ultrasound Doppler data may include information regarding movement of at least a portion of a target object (e.g., movement of blood). For example, ultrasound Doppler data may include information regarding a speed and direction of blood flow. The ultrasound data obtainer 110 may measure the overall speed components of a blood vessel of a designated length by using ultrasound Doppler data obtained by using planewave ultrasound signals.

The processor 120 may generate an ultrasound image by performing scan conversion with respect to ultrasound data obtained by the ultrasound data obtainer 110.

Ultrasound images may include not only a grayscale ultrasound image obtained by scanning a target object in amplitude mode (A mode), brightness mode (B mode), and motion mode (M mode), but also a Doppler image showing movement of the target object. Doppler images may include a blood flow Doppler image showing a blood flow (a.k.a., color Doppler image; referred to hereinafter as a 'C mode image'), a tissue Doppler image showing movement of tissues, and a spectral Doppler image (referred to hereinafter as a 'PW image') showing a moving speed of a target object in a waveform.

Furthermore, the processor 120 may generate a 3D ultrasound image by performing volume rendering with respect to volume data. Furthermore, the processor 120 may further generate an elastic image showing deformation of the target object due to a pressure and also including various texts or graphic information.

The processor 120 obtains blood flow speeds with respect to a plurality of areas of a blood vessel based on ultrasound data acquired by the ultrasound data obtainer 110. The processor 120 may obtain blood flow speed values of a blood flow passing through the centers of a plurality of cross-sections of the blood vessel.

Furthermore, the processor 120 determines stenosis degrees of the plurality of areas based on the obtained blood flow speeds.

The processor 120 may select a reference cross-section from among the plurality of cross-sections of the blood vessel. For example, the processor 120 may select the cross-section corresponding to the slowest blood flow speed from among the plurality of cross-sections of the blood vessel or may select a cross-section based on a user input. Alternatively, the processor 120 may obtain a B mode image or a C mode image by processing obtained ultrasound data and may analyze the B mode image or the C mode image to select a reference cross-section from among the plurality of cross-sections.

Furthermore, the processor 120 may determine stenosis degrees of cross-sections other than a reference cross-section based on the speed of the blood flow passing through the selected reference cross-section.

The term stenosis degree of a cross-section of a blood vessel may refer to a narrowing degree of a blood vessel due to accumulation of plagues on a blood vessel wall. Stenosis degree of a cross-section of a blood vessel may be measured by comparing a reference area to a blood-flowing area of the cross-section. The reference area may refer to an actual area of a blood vessel excluding plagues accumulated on a blood vessel wall. In other words, the processor 120 subtracts a blood-flowing area of a cross-section from a reference area, thereby measuring a ratio of plagues accumulated on a blood vessel wall. The processor 120 according to an embodiment of the present invention may determine an area of a selected reference cross-section as a reference area.

The processor 120 may determine stenosis degrees of a plurality of cross-sections of a blood vessel by comparing speeds of blood flows passing through the plurality of cross-sections. In other words, since mass of a fluid flowing in a tube is constant regardless of the thickness of the tube, the processor 120 may determine a portion corresponding to a relatively fast blood flow speed as a portion corresponding to a relatively narrow cross-section of a blood vessel, that is, a portion corresponding to a relatively high stenosis degree.

The processor 120 may calculate ratios between area of a selected reference cross-section and areas of cross-sections other than the reference cross-section based on ratios between a speed of a blood flow passing through the selected reference cross-section and speeds of the blood flows passing through the other cross-sections other than the selected reference blood flow. The processor 120 may determine stenosis degrees of a plurality of cross-sections based on the calculated ratios.

Furthermore, the processor 120 generates an ultrasound image of a blood vessel that indicates the determined stenosis degrees.

The display 130 displays the image of the blood vessel generated by the processor 120.

The image generated by the processor 120 may include an image in which stenosis degrees of the plurality of the blood vessel are shown by using at least one from among colors, graphs, and geometric shapes.

Furthermore, in the image generated by the processor 120, a C mode image may be displayed in a region inside the blood vessel and a B mode image including colors indicating structure of a blood vessel may be displayed in a region corresponding to a blood vessel wall of the blood vessel.

The display 130 may display not only an ultrasound image generated by the processor 120 but also various information processed by the ultrasound image displaying device 100 via a graphic user interface (GUI).

The ultrasound image displaying device 100 according to an embodiment of the present invention may measure the overall speed components of the blood vessel not only a speed component of a cross-section of the blood vessel by using a planewave ultrasound. And the ultrasound image displaying device 100 may estimate the overall stenosis degrees of the blood vessel based on the measured overall speed components. Furthermore, the ultrasound image displaying device 100 according to an embodiment of the present invention may display a stenosis degree of a blood vessel by using at least one from among colors, graphs, and geometric figures, such that a user may quickly and precisely recognize the stenosis degree of the blood vessel.

Hereinafter, a method used by the ultrasound image displaying device 100 to display an ultrasound image of a blood vessel according to an embodiment of the present invention will be described in detail with reference to FIG. 2.

FIG. 2 is a flowchart showing a method of displaying an ultrasound image according to an embodiment of the present invention.

In an operation S210, the ultrasound image displaying device 100 according to an embodiment of the present invention obtains ultrasound data regarding a target object including a blood vessel.

The ultrasound image displaying device 100 may transmit a planewave ultrasound signal to the target object and may receive an ultrasound echo signal reflected by the target object. The ultrasound image displaying device 100 may obtain the ultrasound data from the ultrasound echo signal received in response to the transmitted planewave ultrasound signal. The ultrasound data obtained by the ultrasound image displaying device 100 may include ultrasound Doppler data, which includes data regarding directions and speeds of movements of tissues inside the target object.

The ultrasound image displaying device 100 according to an embodiment of the present invention may measure the overall speed components of a blood vessel more precisely by obtaining Doppler data based on a planewave ultrasound signal.

An ultrasonography system in the related art that does not use planewave ultrasound signals may provide a color Doppler mode (that is, a C mode image) for a user to recognize a distribution of blood flow speeds with respect to a large area included in a target object. Furthermore, an ultrasonography system in the related art that does not use planewave ultrasound signals may provide a PW image in which Doppler components regarding a set interest region are shown as waveforms so that a user may obtain more precise information regarding blood flow speeds regarding a relatively small area included in a target object.

Meanwhile, the ultrasound image displaying device 100 according to an embodiment of the present invention may obtain Doppler data by using a planewave ultrasound signal of which focal point is at the infinity. Therefore, the ultrasound image displaying device 100 according to an embodiment of the present invention may extract Doppler components with respect to a large area from a plurality of scan lines parallel to one another via a single ultrasound transmission.

In an operation S220, the ultrasound image displaying device 100 according to an embodiment of the present invention obtains, from the ultrasound data obtained in the operation S210, speeds of blood flows passing through a plurality of cross-sections of a blood vessel.

The ultrasound image displaying device 100 detects a blood vessel area from the obtained ultrasound data and may obtain speeds of blood flows passing through the centers of the plurality of cross-sections of the blood vessel.

In an operation S230, the ultrasound image displaying device 100 determines stenosis degrees of the plurality of cross-sections of the blood vessel based on the blood flow speeds obtained in the operation S220.

The ultrasound image displaying device 100 according to an embodiment of the present invention may determine stenosis degrees of a plurality of cross-sections of a blood vessel from the obtained blood flow speeds based on a continuity equation.

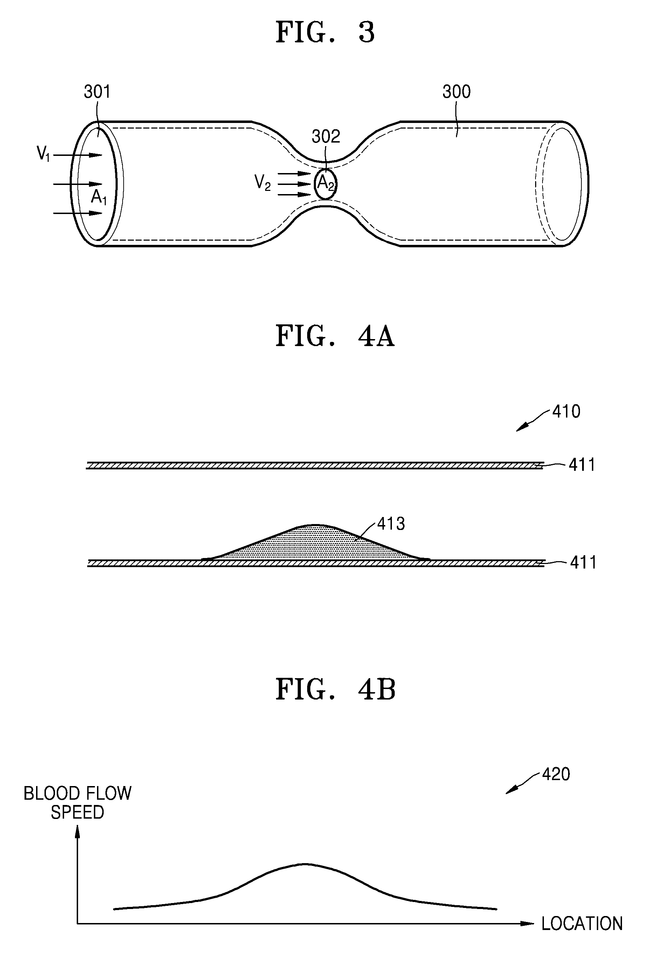

FIG. 3 is a diagram for describing a continuity equation employed in a method of displaying an ultrasound image according to an embodiment of the present invention.

As shown in FIG. 3, the mass of blood flowing in a blood vessel is constant regardless of a thickness of the blood vessel. Therefore, as shown in [Equation 1] below, the mass of blood passing through a cross-section 301 having an area A1 is identical to the mass of blood passing through a cross-section 302 having an area A2.

【Equation 1】

$$A_1 \times V_1 \times T = A_2 \times V_2 \times T$$

In [Equation 1], A1 denotes an area of the cross-section 301 of a blood vessel 300, V1 denotes a speed of a blood flow passing through the cross-section 301, A2 denotes an area of the cross-section 302 of the blood vessel 300, and V2 denotes a speed of a blood flow passing through the cross-section 302. According to [Equation 1], the area of the blood vessel is inversely proportional to the speed of the blood flow.

Therefore, the ultrasound image displaying device 100 according to an embodiment of the present invention may calculate ratios of a plurality of cross-sections of the blood vessel based on ratios of the blood flow speeds obtained in the operation S220 and may determine stenosis degrees of the plurality of cross-sections based on the calculated ratios.

The ultrasound image displaying device 100 may select a reference cross-section from among the plurality of cross-sections of the blood vessel and may determine stenosis degrees of cross-sections other than the selected reference cross-section based on area of the selected reference cross-section.

For example, the ultrasound image displaying device 100 may select a cross-section corresponding to the slowest blood flow from among the plurality of cross-sections or may select a designated cross-section as a reference cross-section based on a user input.

Alternatively, the ultrasound image displaying device 100 may generate a B mode image or a C mode image by processing the ultrasound data obtained in the operation S220 and may analyze the B mode image or the C mode image to select a reference cross-section from among a plurality of cross-sections.

If the thickness of the blood vessel is constant, the cross-section corresponding to the slowest blood flow speed, that is, a cross-section having the largest area, may be assumed to be a cross-section in which no plague is accumulated. The ultrasound image displaying device 100 may select a cross-section corresponding to the slowest maximum speed during a single heartbeat period based on an electrocardiogram (ECG).

The ultrasound image displaying device 100 may analyze a B mode image or a C mode image in order to select a cross-section having the largest area or a cross-section corresponding to the slowest blood flow speed as a reference cross-section. Furthermore, the ultrasound image displaying device 100 may determine a blood vessel area having smaller area compared to the selected reference cross-section as a blood vessel areas in which stenosis degree is formed due to accumulation of plagues.

As presented above, the ultrasound image displaying device 100 may select a reference cross-section from among a plurality of cross-sections.

Therefore, the ultrasound image displaying device 100 may select the reference cross-section from among the plurality of cross-sections and may determine stenosis degrees of cross-sections other than the selected reference cross-section based on the speed of the blood flow passing through the selected reference cross-section. In other words, when the speed of the blood flow passing through a designated cross-section is greater than the speed of the blood vessel passing through a reference cross-section, the ultrasound image displaying device 100 may determine that a stenosis degree occurred in the designated cross-section.

In order to measure a stenosis degree of a blood vessel by using an ultrasonography system, a B mode image of a cross-section of the blood vessel is obtained and a thickness of a blood vessel wall or area of the blood vessel is manually measured by a user in order to diagnose the stenosis degree of the blood vessel.

However, since the ultrasound image displaying device 100 according to an embodiment of the present invention automatically determines a stenosis degree of a blood vessel based on blood flow speeds, a precise measurement may be obtained without deviations from one user to another. Furthermore, in the case of a fiber plague that is echo-poor with respect to a B mode image (it is difficult to recognize a fiber plague in a B mode image), a stenosis degree of a blood vessel is measured by using Doppler components of ultrasound data, and thus, a stenosis degree of a blood vessel based on the fiber plague may be precisely measured.

In an operation S240, the ultrasound image displaying device 100 according to an embodiment of the present invention generates and displays an image of a blood vessel that shows the stenosis degrees determined in the operation S230.

The ultrasound image displaying device 100 may display the overall stenosis degrees of the blood vessel by selecting a reference cross-section from among a plurality of cross-sections and comparing the selected reference cross-section to cross-sections other than the reference cross-section. The ultrasound image displaying device 100 may display stenosis degrees of the plurality of cross-sections by using at least one from among colors, graphs, geometric figures, brightness, texts, and symbols.

For example, as shown in FIG. 4A, the ultrasound image displaying device 100 may generate and display an image 410 which displays a sectional view of a blood vessel. The image 410 generated by the ultrasound image displaying device 100 may include an image showing a plague 413 accumulated on a blood vessel wall 411. In the plague image displayed by the ultrasound image displaying device 100, at least one from among a size of the plaque image, a shape of the plaque image, and a location where the plaque image is displayed in the screen may be modified based on the stenosis degree of the blood vessel determined based on the blood flow speeds.

As another example, as shown in FIG. 4B, the ultrasound image displaying device 100 may provide to a user information regarding a stenosis degree of a blood vessel by generating and displaying a graph 420 showing blood flow speeds. The user may determine a blood vessel area corresponding to a high blood flow speed as an area with a high stenosis degree of the blood vessel based on the blood flow speeds shown in the graph 420.

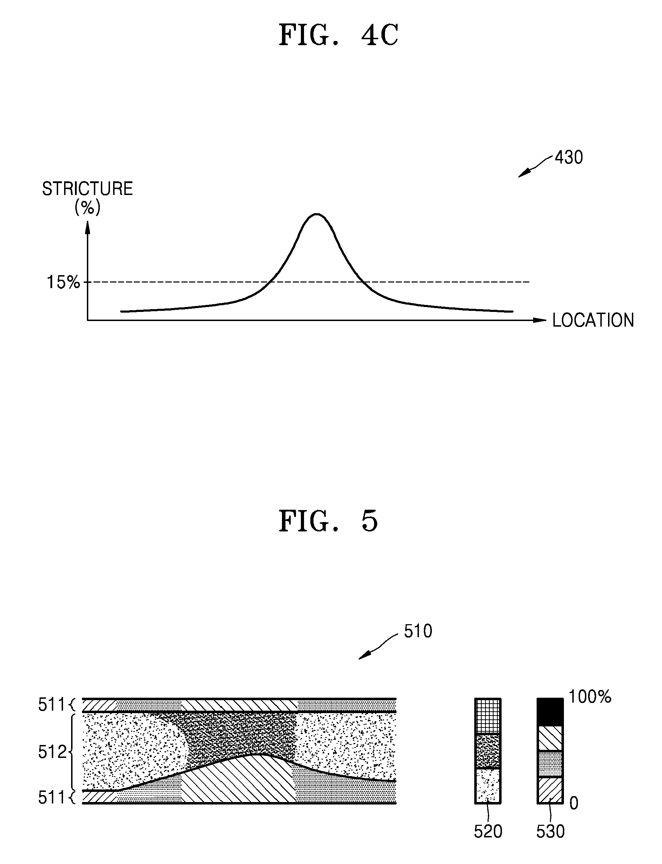

As another example, as shown in FIG. 4C, the ultrasound image displaying device 100 may generate and display an image including a graph 430 showing a stenosis degree of a blood vessel. The ultrasound image displaying device 100 may display a critical value, which may indicate a high risk of a circulatory disease, on the graph 430 showing the stenosis degree of the blood vessel. For example, in FIG. 4C, if an accumulated plague occupies 15% or more of the blood vessel area, a critical value 15% is displayed on the graph 430 for indicating a high risk of a circulatory disease.

Meanwhile, the ultrasound image displaying device 100 according to an embodiment of the present invention may generate and display an image showing a stenosis degree of a blood vessel on at least one from between a B mode image or a C mode image regarding a target object.

FIG. 5 is a diagram showing an example of images of a blood vessel according to an embodiment of the present invention.

As shown in FIG. 5, the ultrasound image displaying device 100 according to an embodiment of the present invention may display a B mode image, may display a C mode image in an area 512 of the B mode image inside a blood vessel, and may display colors showing determined stenosis degrees in an area 511 of the B mode image corresponding to a blood vessel wall of the blood vessel.

The ultrasound image displaying device 100 may display at least one color selected based on a stenosis degree of a blood vessel in the area 511 corresponding to the blood vessel wall. The ultrasound image displaying device 100 may display a color bar 530 displaying at least one color allocated to the area 511 corresponding to the blood vessel wall together with an image 510 showing the blood vessel.

The ultrasound image displaying device 100 may display in the area 512 inside the blood vessel a C mode image to which at least one color selected based on speed and direction of a blood flow is allocated. The ultrasound image displaying device 100 may display a color bar 520 displaying at least one color allocated to the area 512 inside the blood vessel together with the image 510 showing the blood vessel.

As described above, the ultrasound image displaying device 100 according to an embodiment of the present invention may measure the overall speed components of a blood flow through a blood vessel by using an ultrafast Doppler imaging method, thereby allowing a user to observe the stenosis degrees determined based on the speed components. Therefore, the user may improve the speed and accuracy of diagnosing a stenosis degree of a blood vessel by using the ultrasound image displaying device 100 according to an embodiment of the present invention.

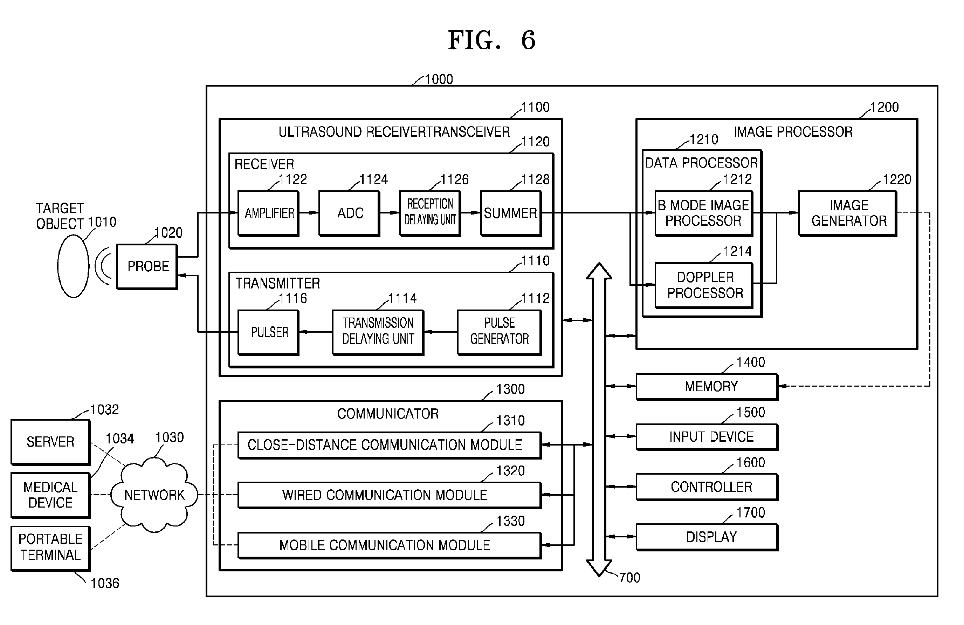

FIG. 6 is a block diagram showing an ultrasonography system using a method and an apparatus for displaying an ultrasound image according to an embodiment of the present invention.

A method of displaying an ultrasound image according to an embodiment of the present invention may be performed by an ultrasonography system 1000 as shown in FIG. 6, and an ultrasound image displaying device according to an embodiment of the present invention may be included in the ultrasonography system 1000 as shown in FIG. 6.

The ultrasound image displaying device 100 of FIG. 1 may perform all or some functions of the ultrasonography system 1000 of FIG. 6 and may include all or some components of the ultrasonography system 1000. In detail, the ultrasound data obtainer 110 and the processor 120 of FIG. 1 may perform all or some functions of a probe 1020, an ultrasound receiver 1100, and an image processor 1200 of FIG. 6, and the display 130 of FIG. 1 may perform all or some functions of a display 1700 of FIG. 6.

The ultrasonography system 1000 according to an embodiment of the present invention may include the probe 1020, the ultrasound receiver 1100, the image processor 1200, a communicator 1300, a memory 1400, an input device 1500, a controller 1600, and a display 1700, which may be connected to one another via a bus 700.

A transmitter 1110 supplies a driving signal to the probe 1020 and includes a pulse generator 1112, a transmission delaying unit 1114, and a pulser 1116. The pulse generator 1112 generates a pulse for forming a transmission ultrasound based on a designated pulse repetition frequency (PRF), and the transmission delaying unit 1114 applies a delay time to the pulse for determining a transmission directionality thereof. Pulses to which delay times are applied correspond to a plurality of piezoelectric vibrators included in the probe 1020, respectively. The pulser 1116 applies a driving signal (or a driving pulse) to the probe 1020 at timings corresponding to the respective pulses to which the delay times are applied.

A receiver 1120 generates an ultrasound image by processing echo signals received from the probe 1020 and may include an amplifier 1122, an analog-digital converter (ADC) 1124, a reception delaying unit 1126, and a summer 1128. The amplifier 1122 amplifies echo signals through respective channels, and the ADC 1124 analog-digital converts the amplified echo signals. The reception delaying unit 1126 delays the digitally-converted echo signals for determining reception directionality thereof, and the summer 1128 generates ultrasound data by summing the echo signals processed by the reception delaying unit 1126.

The image processor 1200 generates and displays an ultrasound image via scan conversion of the ultrasound data generated by the ultrasound receiver 1100.

A B mode image processor 1212 extracts B mode components from the ultrasound data and processes the B mode components. An image generator 1220 may generate an ultrasound image in which signal intensities are shown as brightness based on the B mode components extracted by the B mode image processor 1212.

In the same regard, a Doppler processor 1214 may extract Doppler components from the ultrasound data, and the image generator 1220 may generate a Doppler image in which a movement of a target object is shown as colors or waveforms based on the extracted Doppler components.

The image generator 1220 according to an embodiment of the present invention may generate a 3D ultrasound image by volume-rendering volume data and may also generate an elasticity image in which deformation of a target object 1010 due to a pressure is imaged. Furthermore, the image generator 1220 may display various additional information via texts and/or graphics. Meanwhile, a generated ultrasound image may be stored in the memory 1400.

The communicator 1300 is connected to a network 1030 via a wire or wirelessly and communicates with an external device or a server. The communicator 1300 may be connected to a hospital server or other medical devices in a hospital via picture archiving and communication system (PACS) and may exchange data therewith. Furthermore, the communicator 1300 may perform data communication according to the digital imaging and communications in medicine (DICOM) standard.

The communicator 1300 may transmit and receive data related to diagnosis of a target object, such as ultrasound image, ultrasound data, and Doppler data regarding the target object, via the network 1030 and may also transmit and receive medical images, such as a CT, MRI, and an X-ray obtained by other medical devices. Furthermore, the communicator 1300 may receive information regarding a patient, such as a diagnosis history and a treatment schedule, from a server and utilize the information for diagnosing a target object. Furthermore, the communicator 1300 may perform data communication not only with a server or a medical device in a hospital but also with a portable terminal of a medical expert or a patient.

The communicator 1300 may be connected to the network 1030 via a wire or wirelessly and may exchange data with a server 1032, a medical device 1034, or a portable terminal 1036. The communicator 1300 may include one or more components enabling communication with an external device, e.g., a close-distance communication module 1310, a wired communication module 1320, and a mobile communication module 1330.

The close-distance communication module 1310 refers to a module for a close-distance communication within a designated distance. Examples of close-distance communication techniques according to an embodiment of the present invention includes wireless LAN, Wi-Fi, Bluetooth, Zigbee, Wi-Fi direct), ultra wideband (UWB), infrared data association (IrDA), Bluetooth Low Energy, and near field communication (NFC). However, the present invention is not limited thereto.

The wired communication module 1320 refers to a module for communication using electric signals or optical signals. Examples of wired communication techniques according to an embodiment of the present invention may include a pair cable, a coaxial cable, an optical fiber cable, and an Ethernet cable.

The mobile communication module 1330 transmits and receives wireless signals to and from at least one from among a station, an external terminal, and a server on a mobile communication network. The wireless signals may include voice signals, video call signals, or various types of data for transmitting and receiving text/multimedia messages.

The memory 1400 stores various data processed by the ultrasonography system 1000. For example, the memory 1400 may store input/output medical data related to diagnosis of a target object, such as ultrasound data and ultrasound images, and may also store an algorithm or a program executed in the ultrasonography system 1000.

The memory 1400 may be any of various types of storage media, such as a flash memory, a hard disk drive, or an EEPROM. Furthermore, the ultrasonography system 1000 may operate a web storage or a cloud server for performing a storage function of the memory 1400 on the web.

The input device 1500 refers to a unit via which data for controlling the ultrasonography system 1000 is input from a user. The input device 1500 may include hardware components, such as a keypad, a mouse, a touch panel, a touch screen, a trackball, and a jog switch. However, the present invention is not limited thereto, and the input device 1500 may further include various other input units, such as an ECG monitoring module, a breath monitoring module, a voice recognition sensor, a gesture recognition sensor, a fingerprint recognition sensor, an iris recognition sensor, a depth sensor, and a distance sensor. Furthermore, a touch pad that forms a layered structure with a display panel of the display 1700 may be referred to as a touch screen.

The controller 1600 controls the overall operation of the ultrasonography system 1000. In other words, the controller 1600 may control operations with regard to the probe 1020, the ultrasound receiver 1100, the image processor 1200, the communicator 1300, the memory 1400, and the input device 1500 shown in FIG. 1.

The display 1700 may include at least one from among a liquid crystal display, a thin film transistor-liquid crystal display, an organic light-emitting diode, a flexible display, a 3D display, and an electrophoretic display.

All or some of the probe 1020, the ultrasound receiver 1100, the image processor 1200, the communicator 1300, the memory 1400, the input device 1500, and the controller 1600 may be operated by a software module. However, the present invention is not limited thereto, and all or some of the above-stated components may be operated by hardware. Furthermore, at least some of the ultrasound receiver 1100, the image processor 1200, and the communicator 1300 may be included in the controller 1600. However, the present invention is not limited thereto.

The invention can also be embodied as computer readable codes on a computer readable recording medium. The computer readable recording medium is any data storage device that can store data which can be thereafter read by a computer system. Examples of the computer readable recording medium include read-only memory (ROM), random-access memory (RAM), CD-ROMs, magnetic tapes, floppy disks, optical data storage devices, etc. The computer readable recording medium can also be distributed over network coupled computer systems so that the computer readable code is stored and executed in a distributed fashion.

It should be understood that the exemplary embodiments described therein should be considered in a descriptive sense only and not for purposes of limitation. Descriptions of features or aspects within each embodiment should typically be considered as available for other similar features or aspects in other embodiments.

While one or more embodiments of the present invention have been described with reference to the figures, it will be understood by those of ordinary skill in the art that various changes in form and details may be made therein without departing from the spirit and scope of the present invention as defined by the following claims.

## Claims

1. A method of displaying an ultrasound image, the method comprising:

   obtaining ultrasound data regarding a target object including a blood vessel;
   obtaining, from the ultrasound data, speeds of blood flows passing through a plurality of cross-sections of the blood vessel;
   determining stenosis degrees of the plurality of cross-sections based on the blood flow speeds; and
   generating and displaying an ultrasound image of the blood vessel showing the determined stenosis degrees.

2. The method of claim 1, wherein the obtaining of the ultrasound data comprises:

   transmitting a planewave ultrasound signal to the target object;
   receiving ultrasound echo signals reflected by the target object; and
   obtaining the ultrasound data from the received ultrasound echo signals;

3. The method of claim 1, wherein the obtained ultrasound data comprises ultrasound Doppler data regarding the target object, and
   the obtaining of the speeds of the blood flows is performed with respect to the centers of the plurality of cross-sections of the blood vessel.

4. The method of claim 1, wherein the determining of the stenosis degrees of the plurality of cross-sections comprises:

   selecting a reference cross-section from among the plurality of cross-sections; and
   determining stenosis degrees of the cross-sections other than the selected reference cross-section based on the speed of the blood flow passing through the selected reference cross-section.

5. The method of claim 1, wherein the determining of the stenosis degrees of the plurality of cross-sections comprises:

selecting a reference cross-section from among the plurality of cross-sections;
calculating a ratio between an area of the selected reference cross-section and areas of cross-sections other than the selected reference cross-section based on ratios between the speed of the blood flow passing through the selected reference cross-section and the speeds of the blood flows passing through the cross-sections other than the selected reference cross-section; and
determining stenosis degrees of the plurality of cross-sections based on the calculated ratios.

6. The method of claim 4 or 5, wherein the selected reference cross-section is a cross-section corresponding to the slowest blood flow speed from among the plurality of cross-sections or a cross-section based on a user input.

7. The method of claim 4 or 5, wherein the selecting of the reference cross-section from among the plurality of cross-sections comprises:

generating a B mode image or a C mode image by processing the obtained ultrasound data; and
analyzing the B mode image or the C mode image to select the reference cross-section from among the plurality of cross-sections .

8. The method of claim 1, wherein the generating and displaying of the image of the blood vessel comprises generating an image of the blood vessel that shows the stenosis degrees of the plurality of cross-sections by using at least one from among colors, graphs, and geometric figures.

9. The method of claim 1, wherein the generating and displaying of the image regarding the blood vessel comprises:

generating a B mode image showing the blood vessel;
displaying the B mode image; and
displaying a C mode image in an area of the B mode image inside the blood vessel and displaying colors showing the determined stenosis degrees in an area of the B mode image corresponding to a blood vessel wall of the blood vessel.

10. An ultrasound image displaying device comprising:

an ultrasound data obtainer, which obtains ultrasound data regarding a target object including a blood vessel;
a processor, which obtains speeds of blood flows passing through a plurality of cross-sections of the blood vessel from the ultrasound data, determines stenosis degrees of the plurality of cross-sections based on the blood flow speeds, and generates an ultrasound image of the blood vessel showing the determined stenosis degrees; and
a display, which displays the image of the blood vessel.

11. The ultrasound image displaying device of claim 10, wherein the processor selects a reference cross-section from among the plurality of cross-sections, and
the processor determines stenosis degrees of the cross-sections other than the selected reference cross-section based on a speed of the blood flow passing through the selected reference cross-section.

12. The ultrasound image displaying device of claim 11, wherein the selected cross-section is a cross-section corresponding to the slowest blood flow speed from among the plurality of cross-sections or a cross-section based on a user input.

13. The ultrasound image displaying device of claim 10, wherein the processor generates an image of the blood vessel that shows the stenosis degrees of the plurality of cross-sections by using at least one from among colors, graphs, and geometric figures.

14. The ultrasound image displaying device of claim 10, wherein the processor generates a B mode image of the blood vessel,
the display displays the B mode image, and
the display displays a C mode image in an area of the B mode image inside the blood vessel and displays colors showing the determined stenosis degrees in an area of the B mode image corresponding to a blood vessel wall of the blood vessel.

**15.** A computer readable recording medium having recording thereon a computer program for implementing the method of any of claims 1 through 9.

# FIG. 1

100

ULTRASOUND IMAGE DISPLAYING DEVICE

110

ULTRASOUND
DATA OBTAINER

120

PROCESSOR

130 — DISPLAY

# FIG. 2

START

OBTAIN ULTRASOUND DATA REGARDING
TARGET OBJECT INCLUDING BLOOD VESSEL — S210

CALCULATE, FROM OBTAINED ULTRASOUND DATA,
SPEEDS OF BLOOD FLOW PASSING THROUGH
PLURALITY OF CROSS-SECTIONS OF BLOOD VESSEL — S220

DETERMINE STENOSIS DEGREES OF PLURALITY OF
CROSS-SECTIONS BASED ON BLOOD FLOW SPEEDS — S230

GENERATE AND DISPLAY IMAGE REGARDING BLOOD
VESSEL SHOWING DETERMINED STENOSIS DEGREES — S240

END

# FIG. 3

# FIG. 4A

# FIG. 4B

# FIG. 4C

STRICTURE
(%)

430

15%

LOCATION

# FIG. 5

510

511

512

511

100%

0

520    530

# FIG. 6

TARGET OBJECT 1010

PROBE 1020

1000

## ULTRASOUND RECEIVERTRANSCEIVER 1100

### RECEIVER 1120

AMPLIFIER 1122 → ADC 1124 → RECEPTION DELAYING UNIT 1126 → SUMMER 1128

### TRANSMITTER 1110

PULSER 1116 ← TRANSMISSION DELAYING UNIT 1114 ← PULSE GENERATOR 1112

## IMAGE PROCESSOR 1200

### DATA PROCESSOR 1210

B MODE IMAGE PROCESSOR 1212

DOPPLER PROCESSOR 1214

IMAGE GENERATOR 1220

## COMMUNICATOR 1300

CLOSE-DISTANCE COMMUNICATION MODULE 1310

WIRED COMMUNICATION MODULE 1320

MOBILE COMMUNICATION MODULE 1330

MEMORY 1400

INPUT DEVICE 1500

CONTROLLER 1600

DISPLAY 1700

700

SERVER 1032

MEDICAL DEVICE 1034

PORTABLE TERMINAL 1036

NETWORK 1030

EP 2 886 058 A1

Europäisches
Patentamt

European
Patent Office

Office européen
des brevets

# EUROPEAN SEARCH REPORT

Application Number

EP 14 19 1024

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 98/23211 A1 (DIASONICS ISRAEL LTD [IL]; FRIEDMAN ZVI [IL]; HALMANN MENACHEM [IL]) 4 June 1998 (1998-06-04) * the whole document * | 1-15 | INV. A61B8/06 A61B8/08 |
| X | JP 2007 289588 A (YAMAYA TOMOYUKI) 8 November 2007 (2007-11-08) * the whole document * | 1-8, 10-13,15 | |
| X | CHRISTOPHER B WONG ET AL: "A novel method to quantify carotid artery stenosis by Doppler ultrasound: Using the continuity principle", INTERNATIONAL JOURNAL OF ANGIOLOGY, vol. 19, no. 02, 1 June 2010 (2010-06-01), pages e86-e90, XP055188288, ISSN: 1061-1711, DOI: 10.1055/s-0031-1278371 | 1-7, 10-12,15 | |
| Y | * page e86 - page e87 * | 8,9,13, 14 | |
| X | US 2009/292208 A1 (JEFFREY JR R BROOKE [US] ET AL) 26 November 2009 (2009-11-26) * the whole document * | 1-3,10, 15 | TECHNICAL FIELDS SEARCHED (IPC) A61B |
| Y | US 6 503 202 B1 (HOSSACK JOHN A [US] ET AL) 7 January 2003 (2003-01-07) * column 5, line 60 - column 6, line 53 * * column 9, line 44 - column 11, line 13 * * column 14, line 3 - line 21; figures * | 8,9,13, 14 | |

The present search report has been drawn up for all claims

| Place of search | Date of completion of the search | Examiner |
|---|---|---|
| Munich | 8 May 2015 | Küster, Gunilla |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................................
& : member of the same patent family, corresponding
document

EPO FORM 1503 03.82 (P04C01)

**ANNEX TO THE EUROPEAN SEARCH REPORT
ON EUROPEAN PATENT APPLICATION NO.**                    EP 14 19 1024

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2015

| Patent document cited in search report | | Publication date | Patent family member(s) | | Publication date |
|---|---|---|---|---|---|
| WO 9823211 | A1 | 04-06-1998 | AU | 5132898 A | 22-06-1998 |
| | | | EP | 0955888 A1 | 17-11-1999 |
| | | | JP | 2001506517 A | 22-05-2001 |
| | | | WO | 9823211 A1 | 04-06-1998 |
| JP 2007289588 | A | 08-11-2007 | NONE | | |
| US 2009292208 | A1 | 26-11-2009 | NONE | | |
| US 6503202 | B1 | 07-01-2003 | NONE | | |

EPO FORM P0459

For more details about this annex : see Official Journal of the European Patent Office, No. 12/82

**EP 2 886 058 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020130158675